# EUROPEAN PATENT APPLICATION

(11) **EP 4 393 537 A1**
(43) Date of publication of application: **03.07.2024**
(21) Application number: 23834565.6
(22) Date of filing: 05.06.2023
(51) Int. Cl.: A61M 60/81, A61M 60/139, A61M 60/216, A61M 60/802, A61M 60/857, F04D 29/047, F04D 29/057

(54) **DRIVING APPARATUS AND BLOOD PUMP**

(30) Priority: 08.07.2022 CN 202210799477
(71) Applicant: Shenzhen Core Medical Technology Co., Ltd., Shenzhen, Guangdong 518000 (CN)
(72) Inventor: ZHU, Yicheng, Shenzhen, Guangdong 518000 (CN); YU, Shunzhou, Shenzhen, Guangdong 518000 (CN)
(74) Representative: Appleyard Lees IP LLP
(86) International application number: PCT/CN2023/098342
(87) International publication number: WO 2024/007791

(57) **Abstract**

Provided are a blood pump (1) and a driving apparatus (20). The driving apparatus (20) comprises a housing assembly (100), a rotating shaft (210), and a rotating head (220) fixedly connected to the rotating shaft (210). The housing assembly (100) is provided with a mounting hole (102). The mounting hole (102) is provided with a concave spherical wall (103). The rotating shaft (210) is provided with a connecting section (211) located outside the housing assembly (100) and configured for being connected to an impeller (40). The rotating head (220) is rotatably arranged in the mounting hole (102) and is provided with a convex spherical surface (222). The convex spherical surface (222) protrudes in a direction away from the connecting section (211) along the axis of the rotating shaft (210), and can abut against the concave spherical wall (103).

## Description

### CROSS-REFERENCE TO RELATED APPLICATIONS

This application claims priority to Chinese patent application No. 202210799477.5, filed with the China Patent Office on July 8, 2022, the entire content of which is hereby incorporated by reference in its entirety.

### TECHNICAL FIELD

The present application relates to the technical field of medical devices, and in particular to a driving device and a blood pump having the same.

### BACKGROUND

An intravascular blood pump is a blood pumping device that can be inserted into a patient's heart through the patient's blood vessels. The intravascular blood pump is placed in an opening of a heart valve, so that blood can flow through the blood pump and into an arterial blood vessel. The blood pump includes a driving portion and an impeller. A driving shaft of the driving portion is connected to the impeller to drive the impeller to rotate. However, since the driving portion of the intravascular blood pump is very small in size, internal parts thereof are also very small and are difficult to assemble, which directly affects production efficiency and yield of the blood pump.

### SUMMARY

Based on this, the present application provides a driving device and a blood pump with relatively low assembly difficulty.

An embodiment of a first aspect of the present application provides a driving device configured to drive an impeller to rotate, the driving device including:
a housing assembly provided with a mounting hole, the mounting hole being provided with a concave spherical wall;
a rotating component including a rotating shaft and a rotating head fixedly connected to the rotating shaft, the rotating shaft being rotatably mounted on the housing assembly, and the rotating shaft being provided with a connecting section located outside the housing assembly and configured to be connected to the impeller, the rotating head being rotatably provided in the mounting hole, the rotating head being provided with a convex spherical surface, the convex spherical surface protruding in a direction away from the connecting section along an axis of the rotating shaft, and the convex spherical surface being capable of abutting against the concave spherical wall;
a rotor fixedly connected to the rotating shaft; and
a stator configured to drive the rotor to rotate, a magnetic force forming between the rotor and the stator, and the magnetic force configured to enable the convex spherical surface to abut against the concave spherical wall.

An embodiment of a second aspect of the present application provides a blood pump, including an impeller and a driving device, the driving device including:
a housing assembly provided with a mounting hole, the mounting hole being provided with a concave spherical wall;
a rotating component including a rotating shaft and a rotating head fixedly connected to the rotating shaft, the rotating shaft being rotatably mounted on the housing assembly, and the rotating shaft being provided with a connecting section located outside the housing assembly and fixedly connected to the impeller, the rotating head being rotatably provided in the mounting hole, the rotating head being provided with a convex spherical surface, the convex spherical surface protruding in a direction away from the connecting section along an axis of the rotating shaft, and the convex spherical surface being capable of abutting against the concave spherical wall;
a rotor fixedly connected to the rotating shaft; and
a stator configured to drive the rotor to rotate, a magnetic force forming between the rotor and the stator, and the magnetic force configured to enable the convex spherical surface to abut against the concave spherical wall.

Details of one or more embodiments of the present application are set forth in the following accompanying drawings and descriptions. Other features, objectives, and advantages of the present application will become obvious with reference to the specification, the accompanying drawings, and the claims.

### BRIEF DESCRIPTION OF THE DRAWINGS

In order to more clearly illustrate the technical solutions in embodiments of the present application, the accompanying drawings to be used in the description of the embodiments or the prior art will be briefly introduced below. It is apparent that, the accompanying drawings in the following description are only some embodiments of the present application, and other accompanying drawings can be obtained by those of ordinary skill in the art from the provided accompanying drawings without creative efforts.
FIG. 1 is a perspective view of a blood pump according to a first embodiment.
FIG. 2 is a partial cross-sectional view of the blood pump shown in FIG. 1.
FIG. 3 is an enlarged view of a part I in FIG. 2.
FIG. 4 is an exploded view of the blood pump shown in FIG. 1 with a catheter omitted.
FIG. 5 is a schematic view of a shaft sleeve of the blood pump shown in FIG. 1.
FIG. 6 is a perspective view of a rotating component of the blood pump shown in FIG. 1.
FIG. 7 is a perspective view of a rotating head of the rotating component shown in FIG. 6.
FIG. 8 is a perspective view of a rotor and a stator of the blood pump shown in FIG. 1.
FIG. 9 is another perspective view of the rotor and the stator shown in FIG. 8.
FIG. 10 is a perspective view of a rotating head of a driving device of a blood pump according to a second embodiment.
FIG. 11 is a cross-sectional view of the rotating head and a shaft sleeve of the driving device of the blood pump according to the second embodiment.
FIG. 12 is a partial cross-sectional view of a blood pump according to a third embodiment.
FIG. 13 is a perspective view of a stator of a driving device of a blood pump according to a fourth embodiment.
FIG. 14 is a perspective view of a stator and a rotor of a driving device of a blood pump according to a fifth embodiment, and
FIG. 15 is a partial cross-sectional view of a blood pump according to a sixth embodiment.

### DETAILED DESCRIPTION OF THE EMBODIMENTS

In order to make the objectives, technical solutions, and advantages of the present application more clearly understood, the present application is described in further detail below in conjunction with the accompanying drawings and embodiments. It should be understood that specific embodiments described herein are intended only to interpret the present application and not intended to limit the present application.

It should be noted that when one element is referred to as "fixed to" or "arranged on" another element, it may be directly disposed on the another element or an intermediate element may exist. When one element is considered to be "connected to" another element, it may be directly connected to the another element or indirectly connected to the another element.

In addition, the terms "first" and "second" are used for descriptive purposes only, which cannot be construed as indicating or implying a relative importance, or implicitly specifying the number of the indicated technical features. Thus, the features defined with "first" and "second" may explicitly or implicitly include one or more features. In the description of the present application, "a plurality of" means two or more, unless specifically stated otherwise.

In order to illustrate the technical solutions of the present application, description will be made below with reference to specific drawings and embodiments.

Herein, "a proximal end" is defined as an end adjacent to an operator; and "a distal end" is defined as an end away from the operator.

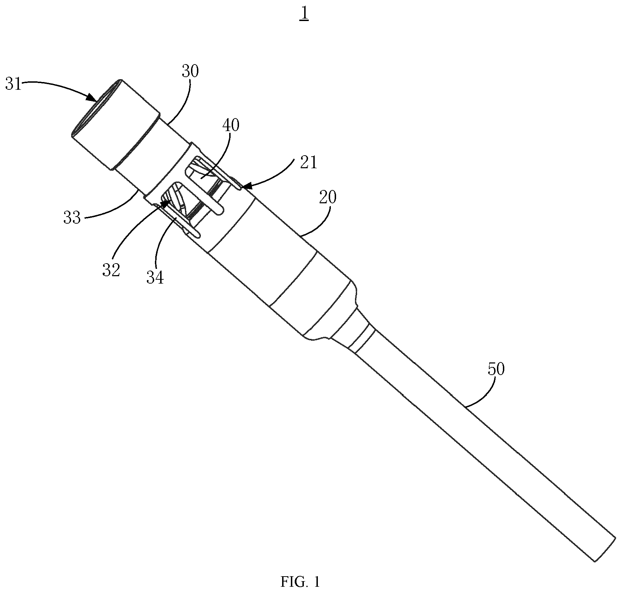

Referring to FIG. 1, a blood pump 1 according to an embodiment includes a driving device 20, a cannula assembly 30, an impeller 40, and a catheter 50. The cannula assembly 30 is connected to a distal end of the driving device 20. The catheter 50 is connected to a proximal end of the driving device 20. The impeller 40 is rotatably provided in the cannula assembly 30. The impeller 40 is in transmission connection the driving device 20. The driving device 20 can drive the impeller 40 to rotate, so as to realize a blood pumping function of the blood pump 1.

Specifically, the cannula assembly 30 is provided with a liquid inlet 31 and a liquid outlet 32. The liquid outlet 32 is closer to the driving device 20 than the liquid inlet 31. That is, the liquid outlet 32 is located at a proximal end of the cannula assembly 30, and the liquid inlet 31 is located at a distal end of the cannula assembly 30. Specifically, the liquid outlet 32 is located on a sidewall of the cannula assembly 30. In an embodiment, the cannula assembly 30 extends through a heart valve, such as an aortic valve, while the liquid inlet 31 is located within a heart, and the liquid outlet 32 and the driving device 20 are located in a blood vessel outside the heart, such as an aorta. When the impeller 40 rotates, blood flows into the cannula assembly 30 from the liquid inlet 31, and then flows out of the cannula assembly 30 from the liquid outlet 32 to enter the blood vessel such as the aorta.

In some embodiments, the cannula assembly 30 includes a tube body 33 and a plurality of spaced inserting sheets 34 extending from one end of the tube body 33 along an axis of the tube body 33, and the liquid outlet 32 is formed between two adjacent inserting sheets 34. The driving device 20 is provided with sinking grooves 21, and ends of the inserting sheets 34 away from the tube body 33 are accommodated in the sinking grooves 21. In the illustrated embodiment, numbers of the sinking grooves 21 and the inserting sheets 34 are equal, which form a one-to-one correspondence relationship. For a blood pump in which an axial length of the impeller 40 is short and a liquid guiding surface is formed at the distal end of the driving device 20 to ensure a hydraulic effect at the liquid outlet 32, the distal end of the driving device 20 is accommodated in the cannula assembly 30, and the liquid guiding surface is located in the cannula assembly 30. In order to prevent an excessively large radial diameter of the driving device 20 and also ensure the hydraulic effect at the liquid outlet 32, a tube wall at an end of the cannula assembly 30 configured to sleeve with the driving device 20 may be very thin, which affects the connection strength between the cannula assembly 30 and the driving device 20. However, by use of the above inserting sheets 34, the inserting sheets 34 may have greater thicknesses, so that the connection strength between the cannula assembly 30 and the driving device 20 is greater.

The catheter 50 butts an end of the driving device 20 away from the cannula assembly 30. The catheter 50 is configured to accommodate various supply lines. The supply lines may be, such as, a flushing line configured to introduce flushing fluid into the driving device 20, a wire configured to supply power to the driving device 20, a supporting member configured to support the catheter 50.

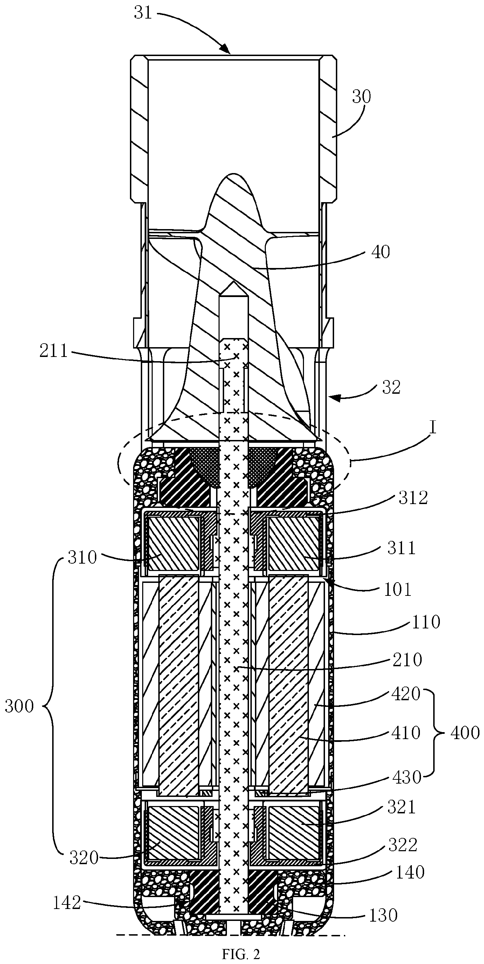

Referring to FIG. 2, FIG. 3, and FIG. 4, the driving device 20 includes a housing assembly 100, a rotating component 200, a rotor 300, and a stator 400.

A distal end of the housing assembly 100 is fixedly connected to the cannula assembly 30, and a proximal end of the housing assembly 100 is fixedly connected to the catheter 50. The housing assembly 100 is provided with a receiving cavity 101 and a mounting hole 102 in communication with the receiving cavity 101. The mounting hole 102 is located at the distal end of the housing assembly 100. The receiving cavity 101 is in communication with the cannula assembly 30 through the mounting hole 102. In this way, the flushing fluid introduced into the receiving cavity 101 from the flushing line can flow out of the housing assembly 100 from the mounting hole 102, enter the cannula assembly 30, and flow out from the liquid outlet 32.

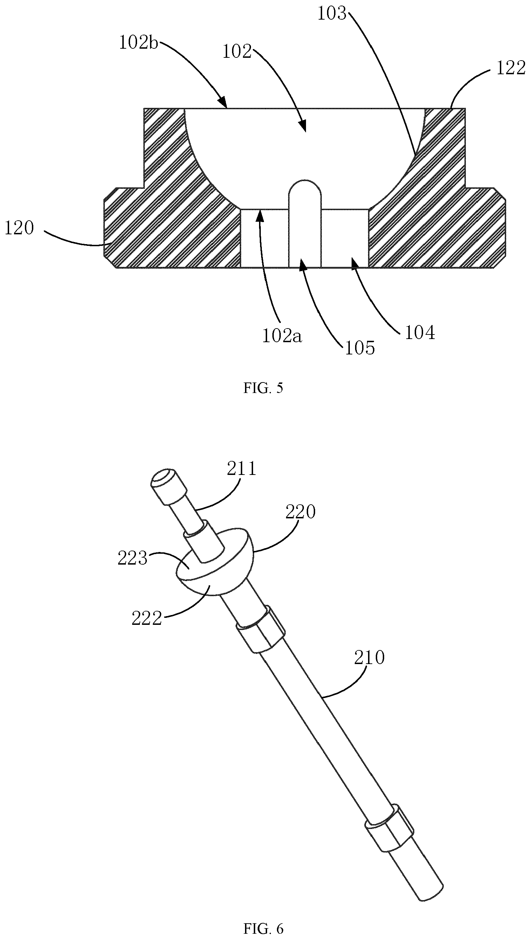

Referring to FIG. 5 together, the mounting hole 102 is provided with a concave spherical wall 103. The mounting hole 102 is provided with a first opening 102a and a second opening 102b opposite to the first opening 102a. The first opening 102a is in communication with the receiving cavity 101, and the second opening 102b is in communication with the outside. Specifically, the second opening 102b is located at the distal end of the housing assembly 100 and is in communication with the cannula assembly 30. A diameter of the mounting hole 102 gradually increases from the first opening 102a to the second opening 102b. In the illustrated embodiment, the concave spherical wall 103 extends from the first opening 102a of the mounting hole 102 to the second opening 102b. The housing assembly 100 is further provided with a communication hole 104 in communication with the mounting hole 102 and the receiving cavity 101, and the diameter of the mounting hole 102 gradually increases in a direction away from the communication hole 104. Therefore, the flushing fluid entering the receiving cavity 101 can enter the mounting hole 102 from the communication hole 104. Specifically, the first opening 102a of the mounting hole 102 is also an opening of the communication hole 104.

Specifically, the housing assembly 100 includes a housing 110 and a shaft sleeve 120. The housing 130 is substantially cylindrical. A distal end of the housing 130 is fixedly connected to the cannula assembly 30, and a proximal end of the housing 130 is fixedly connected to the catheter 50. The receiving cavity 101 is provided in the housing 110. The housing 110 is further provided with a communication port 111 in communication with the receiving cavity 101. The communication port 111 is located at the distal end of the housing 110. That is, the communication port 111 is located at an end of the housing 110 adjacent to the impeller 40 or the cannula assembly 30. The shaft sleeve 120 is mounted in the receiving cavity 101, and the mounting hole 102 is provided in the shaft sleeve 120. The shaft sleeve 120 is provided with an outer surface 122 coplanar with a plane where the communication port 111 is located. A plane where the second opening 102b of the mounting hole 102 is located is coplanar with the outer surface 122 of the shaft sleeve 120. The communication hole 104 is also provided in the shaft sleeve 120. Specifically, the shaft sleeve 120 and the housing 110 may be fixedly connected by bonding, welding, etc. Alternatively, the shaft sleeve 120 and the housing 110 are an integrally formed structure.

Referring to FIG. 6 together, the rotating component 200 is rotatably mounted on the housing assembly 100. The rotating component 200 is partially accommodated in the receiving cavity 101, partially located in the mounting hole 102, and partially located outside the receiving cavity 101 and the mounting hole 102 to be fixedly connected to the impeller 40. The rotating component 200 can drive the impeller 40 to rotate. The rotating component 200 includes a rotating shaft 210 and a rotating head 220 fixedly connected to the rotating shaft 210.

The rotating shaft 210 is rotatably mounted on the housing assembly 100. The rotating shaft 210 is provided with a connecting section 211 configured for being connected to the impeller 40. The connecting section 211 is located outside the housing assembly 100. Specifically, the rotating shaft 210 rotatably extends through the communication hole 104. A maximum diameter of the rotating shaft 210 is greater than a diameter of the communication hole 104 and less than a diameter of the second opening 102b of the mounting hole 102, so that the rotating component 200 can be assembled to the housing assembly 100 in a direction from the second opening 102b of the mounting hole 102 to the first opening 102a.

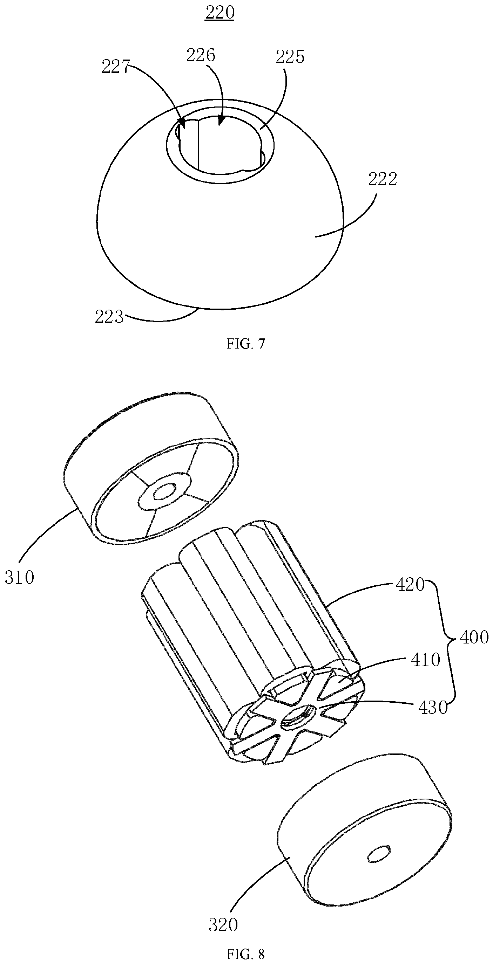

Referring to FIG. 7 together, the rotating head 220 is rotatably provided in the mounting hole 102. The rotating head 220 is provided with a convex spherical surface 222. The convex spherical surface 222 protrudes in a direction away from the connecting section 211 along an axis of the rotating shaft 210, and the convex spherical surface 222 can abut against the concave spherical wall 103. A shape and a size of the convex spherical surface 222 are adapted to those of the concave spherical wall 103. In the illustrated embodiment, the rotating head 220 is substantially in a shape of a dome. An axis of the rotating head 220 coincides with the axis of the rotating shaft 210. A width of the rotating head 220 in a direction perpendicular to an axial direction of the rotating shaft 210 is less than the diameter of the second opening 102b of the mounting hole 102.

Specifically, the rotating head 220 is further provided with a bottom surface 223 connected to the convex spherical surface 222, and the bottom surface 223 is provided towards the impeller 40. When the convex spherical surface 222 abuts against the concave spherical wall 103, the bottom surface 222 is coplanar with the plane where the second opening 102b of the mounting hole 102 is located. In other words, the bottom surface 223 of the rotating head 220 is coplanar with the outer surface 122 of the shaft sleeve 120. The bottom surface 223 is circular. A diameter of the bottom surface 223 is less than the diameter of the second opening 102b of the mounting hole 102. A ratio of a diameter of the first opening 102a of the mounting hole 102 to the diameter of the bottom surface 223 of the rotating head 220 is 0.3 to 0.6. If the diameter of the first opening 102a of the mounting hole 102 is excessively large, a contact area between the convex spherical surface 222 of the rotating head 220 and the concave spherical wall 103 of the mounting hole 102 will be reduced (resulting in a reduction in a force-bearing area), and wear of the convex spherical surface 222 and the concave spherical wall 103 will be increased. If the diameter of the first opening 102a of the mounting hole 102 is excessively small, an amount of the flushing fluid entering the mounting hole 102 from the first opening 102a will be affected. On the one hand, the flushing fluid entering the mounting hole 102 is configured to prevent the blood in the cannula assembly 30 from entering the receiving cavity 101. On the other hand, the flushing fluid enters between the convex spherical surface 222 and the concave spherical wall 103 to provide lubrication, so as to reduce a friction coefficient between the rotating head 220 and the concave spherical wall 103 of the mounting hole 102. The above ratio enables the convex spherical surface 222 of the rotating head 220 and the concave spherical wall 103 of the mounting hole 102 to have less wear and also have a suitable flow rate of the flushing fluid.

In order to prevent the blood in the cannula assembly 30 from entering the mounting hole 102 through the second opening 102b of the mounting hole 102, a gap between a hole wall of the mounting hole 102 at the second opening 102b and the rotating head 220 is preferably less than or equal to 2 microns. In order to ensure a reasonable flow rate and velocity of the flushing fluid in the second opening 102b of the mounting hole 102, a difference between the diameter of the communication hole 104 and a diameter of a portion of the rotating shaft 210 located within the communication hole 104 is greater than 4 microns.

In the illustrated embodiment, a hole wall of the communication hole 104 is partially recessed to form a drainage groove 105 extending along an axis of the communication hole 104, and the drainage groove 105 is in communication with both the receiving cavity 101 and the mounting hole 102, which can increase the flow rate of the flushing fluid flowing into the mounting hole 102. Compared with the manner of directly increasing the diameter of the communication hole 104, the manner of providing the drainage groove 105 in the hole wall of the communication hole 104 to increase the flow rate of the flushing fluid enables the concave spherical wall 103 of the mounting hole 102 to have a larger area, so that the concave spherical wall 103 and the convex spherical surface 222 of the rotating head 220 have a larger contact area, so as to share pressure of the rotating head 220 against the concave spherical wall 103, thereby reducing the wear of the concave spherical wall 103 and the rotating head 220.

In the illustrated embodiment, the rotating head 220 is further provided with a top surface 225, the top surface 225 and the bottom surface 223 are spaced apart along the axis of the rotating shaft 210and provided in parallel, a diameter of the top surface 225 is less than that of the bottom surface 223, and the convex spherical surface 222 connects the top surface 225 and the bottom surface 223, so that the top surface 225, the bottom surface 223, and the convex spherical surface 222 cooperatively form a dome structure. The rotating shaft 210 is provided through the top surface 225 and the bottom surface 223 of the rotating head 220, and the axis of the rotating shaft 210 is perpendicular to the top surface 225 and the bottom surface 223 of the rotating head 220. The diameter of the top surface 225 of the rotating head 220 is less than the diameter of the first opening 102a of the mounting hole 102, so that the rotating head 220 is partially located outside the mounting hole 102, in other words, the rotating head 220 is partially located in the communication hole 104, so as to ensure the contact area between the convex spherical surface 222 of the rotating head 220 and the concave spherical wall 103 of the mounting hole 102 as much as possible, and prevent the contact between the top surface 225 of the rotating head 220 and the concave spherical wall 103 of the mounting hole 102 due to an angle formed by a junction between the top surface 225 of the rotating head 220 and the convex spherical surface 222, thereby effectively reducing the wear of the rotating head 220 and the concave spherical wall 103 of the mounting hole 102.

In the illustrated embodiment, the rotating shaft 210 is further provided with a limiting surface 213, and the top surface 225 of the rotating head 220 abuts against the limiting surface 213, so as to position the rotating head 220 on the rotating shaft 210, which facilitates mounting and positioning of the rotating head 220. Specifically, the rotating head 220 is provided with a through hole 226 extending through the top surface 225 and the bottom surface 223 of the rotating head 220. A center axis of the through hole 226 is perpendicular to both the top surface 225 and the bottom surface 223. The center axis of the through hole 226 passes through a center of a circle where the top surface 225 is located and also passes through a center of a circle where the bottom surface 223 is located. The rotating shaft 210 extends through the through hole 226. A hole wall of the through hole 226 is provided with a glue groove 227. The glue groove 227 is configured to be filled with an adhesive material configured to bond and fix the rotating head 220 and the rotating shaft 210. Specifically, the glue groove 227 extends from the top surface 225 of the rotating head 220 toward the bottom surface 223. It should be understood that, in other embodiments, the rotating head 220 may alternatively be fixedly connected to the rotating shaft 210 by welding, or the rotating head 220 and the rotating shaft 210 may be an integrally formed structure.

The rotor 300 is fixedly connected to the rotating shaft 210, and the stator 400 can drive the rotor 300 to rotate, so that the rotor 300 can drive the rotating shaft 210 to rotate and the impeller 40 can rotate along with the rotating shaft 210. A magnetic force is formed between the rotor 300 and the stator 400, and the magnetic force enables the convex spherical surface 222 to abut against the concave spherical wall 103.

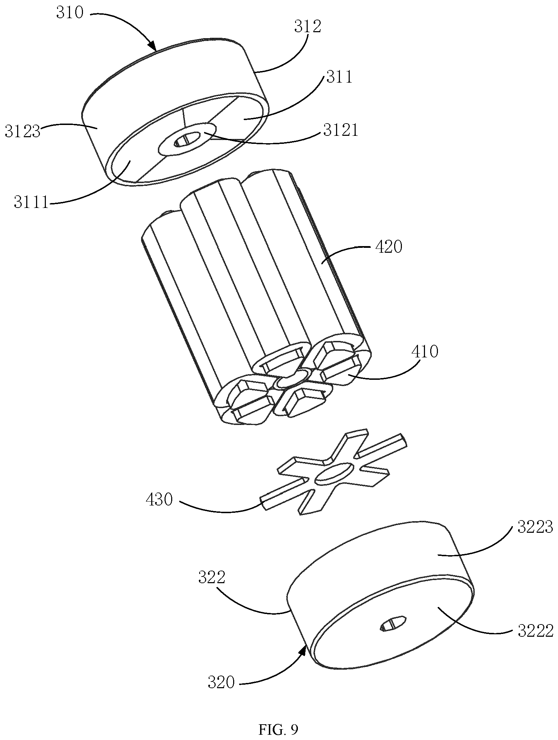

Referring to FIG. 2, FIG. 4, FIG. 8, and FIG. 9 together, in the illustrated embodiment, the rotor 300 includes a first rotor unit 310 and a second rotor unit 320 both fixedly connected to the rotating shaft 210. Both the first rotor unit 310 and the second rotor unit 320 are accommodated in the receiving cavity 101. The rotating head 220, the first rotor unit 310, the stator 400, and the second rotor unit 320 are arranged sequentially along the axis of the rotating shaft 210. Specifically, the first rotor unit 310 is located between the shaft sleeve 120 and the stator 400. The stator 400 can drive the first rotor unit 310 and the second rotor unit 320 to rotate. A first attractive force is formed between the stator 400 and the first rotor unit 310, a second attractive force is formed between the stator 400 and the second rotor unit 320, and the first attractive force is greater than the second attractive force. The convex spherical surface 222 abuts against the concave spherical wall 103 under a combined action of the first attractive force and the second attractive force. That is, the rotor 300 is subjected to a magnetic force of the stator 400, which is a resultant force of the first attractive force and the second attractive force and is oriented in a direction away from the connecting section 211 of the rotating shaft 210, so that the rotating component 200 is subjected to a force in a direction away from the connecting section 211 of the rotating shaft 210. The force causes the convex spherical surface 222 to be abut against the concave spherical wall 103.

Specifically, the first rotor unit 310 and the second rotor unit 320 are both magnetic, and the stator 400 can generate a rotating magnetic field to drive the first rotor unit 310 and the second rotor unit 320 to rotate. In an embodiment, when distances of spacings are equal, an attractive force between the first rotor unit 310 and the stator 400 is equal to that between the second rotor unit 320 and the stator 400. The first rotor unit 310 and the second rotor unit 320 have a same structure. In the illustrated embodiment, a distance between the first rotor unit 310 and the stator 400 is less than that between the second rotor unit 320 and the stator 400, so that the first attractive force is greater than the second attractive force.

The first rotor unit 310 includes a first magnetic member 311, and the first magnetic member 311 is fixedly connected to the rotating shaft 210. The first magnetic member 311 is an annular Halbach array magnet. The first magnetic member 311 includes a plurality of first magnetic units 3111. Each first magnetic unit 3111 is in a shape of a sector ring. The plurality of first magnetic units 3111 are arranged in a circle around the rotating shaft 210, so that the first magnetic member 311 forms a ring structure. Specifically, four, six, eight, ten, etc. first magnetic units 3111 are provided.

The first rotor unit 310 further includes a first flywheel 312 is fixedly connected to the rotating shaft 210, and the first magnetic member 311 is fixedly connected to the first flywheel 312. By providing the first flywheel 312, the connection strength between the first magnetic member 311 and the rotating shaft 210 can be enhanced, and the shaking of the rotating shaft 210 during rotation can also be reduced, so that the whole rotating shaft 210 is more stable during rotation.

Specifically, the first flywheel 312 includes a first built-in tube 3121, a first disc-shaped portion 3122, and a first outer ring wall 3123. Both the first built-in tube 3121 and the first outer ring wall 3123 are circular tubular structures, and the first disc-shaped portion 3122 is an annular disk structure. Both the first built-in tube 3121 and the first outer ring wall 3123 are fixedly connected to the first disc-shaped portion 3122. The first outer ring wall 3123 is arranged around the first disc-shaped portion 3122, the first built-in tube 3121 and the first outer ring wall 3123 are arranged coaxially, and the rotating shaft 210 is arranged in the first built-in tube 3121 and fixedly connected to the first built-in tube 3121. A first mounting cavity is formed between the first built-in tube 3121 and the first outer ring wall 3123. The first magnetic member 311 is accommodated in the first mounting cavity. The shape of the first mounting cavity is adapted to the first magnetic member 311 to facilitate the mounting and positioning of the first magnetic member 311. This arrangement enables the first flywheel 312 to limit the first magnetic member 311, which not only facilitates the mounting of the first magnetic member 311, but also enables the combination of the first magnetic member 311 and the first flywheel 312 to be more stable.

It should be noted that the first flywheel 312 is not limited to the above structure. In some embodiments, the first flywheel 312 does not have the first outer ring wall 3123. In some embodiments, the first flywheel 312 does not have the first outer annular wall 3123 and the first built-in tube 3121. In this case, the rotating shaft 210 is fixedly provided in a center of the first disc-shaped portion 3122. Compared with the first flywheel 312 having only the first disc-shaped portion 3122, the first built-in tube 3121 is provided, so that the first flywheel 312 can be more stably connected to the rotating shaft 210.

The second rotor unit 320 includes a second magnetic member 321 fixedly connected to the rotating shaft 210. Specifically, the second magnetic member 321 is an annular Halbach array magnet. A structure of the second magnetic member 321 is substantially the same as that of the first magnetic member 311. The second magnetic member 321 includes a plurality of second magnetic units 3211. Each second magnetic unit 3211 is in the shape of a sector ring. The plurality of second magnetic units 3211 are arranged in a circle around the rotating shaft 210, so that the second magnetic member 321 forms a ring structure. Specifically, four, six, eight, ten, etc. second magnetic units 3211 are provided.

The second rotor unit 320 further includes a second flywheel 322 is fixedly connected to the rotating shaft 210, and the second magnetic member 321 is fixedly connected to the second flywheel 322. By providing the first flywheel 312, the connection strength between the second magnetic member 321 and the rotating shaft 210 can be enhanced, and shaking of the rotating shaft 210 during the rotation can also be reduced, so that the whole rotating shaft 210 is more stable during the rotation.

The second flywheel 322 includes a second built-in tube 3221, a second disc-shaped portion 3222 and a second outer ring wall 3223. Both the second built-in tube 3221 and the second outer ring wall 3223 have circular tubular structures, and the second disc-shaped portion 3222 has an annular disk structure. Both the second built-in tube 3221 and the second outer ring wall 3223 are fixedly connected to the second disc-shaped portion 3222. The second outer ring wall 3223 is arranged around the second disc-shaped portion 3222, the second built-in tube 3221 and the second outer ring wall 3223 are arranged coaxially, and the rotating shaft 210 extends through the second built-in tube 3221 and fixedly connected to the second built-in tube 3221. A second mounting cavity is formed between the second built-in tube 3221 and the second outer ring wall 3223. The second magnetic member 321 is accommodated in the second mounting cavity. A shape of the second mounting cavity is adapted to the second magnetic member 321 to facilitate the mounting and positioning of the second magnetic member 321. Such arrangement enables the second flywheel 322 to limit the second magnetic member 321, which not only facilitates the installation of the second magnetic member 321, but also enables the combination of the second magnetic member 321 and the second flywheel 322 to be more stable.

It should be noted that the second flywheel 322 is not limited to the above structure. In some embodiments, the second flywheel 322 does not have the second outer ring wall 3223. In some embodiments, the second flywheel 322 does not have the second outer ring wall 3223 and the second built-in tube 3221. In this case, the rotating shaft 210 is fixedly provided in a center of the second disc-shaped portion 3222. Compared with the second flywheel 322 having only the second disc-shaped portion 3222, the second built-in tube 3221 is provided, so that the second flywheel 322 can be more stably connected to the rotating shaft 210.

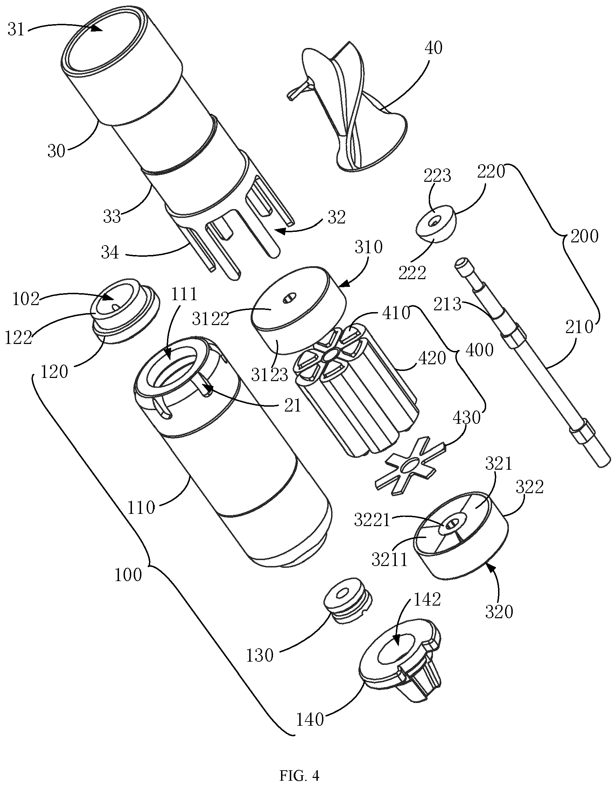

Referring to FIG. 2 and FIG. 4, in some embodiments, the stator 400 includes a plurality of magnetic cores 410 and a plurality of coils 420, the plurality of magnetic cores 410 are spaced apart along a circle, and the plurality of coils 420 are wound around the plurality of magnetic cores 410, respectively. Specifically, an extending direction of each magnetic core 410 is consistent with an extending direction of the rotating shaft 210. The rotating shaft 210 rotatably extends through the stator 400, and the plurality of magnetic cores 410 are provided around the rotating shaft 210.

In the illustrated embodiment, the magnetic core 410 is substantially columnar, and a size of the cross-section of the magnetic core 410 remains constant in the extending direction of the magnetic core 410. The first rotor unit 310 and the second rotor unit 320 are provided adjacent to both ends of the magnetic core 410, respectively. More specifically, the first magnetic member 311 of the first rotor unit 310 is adjacent to one end of the magnetic core 410, and the second magnetic member 321 of the second rotor unit 320 is adj acent to the other end of the magnetic core 410, so that the magnetic core 410 can be magnetically coupled with the first magnetic member 311 and the second magnetic member 321 simultaneously, so that the stator 400 can simultaneously drive the first rotor unit 310 and the second rotor unit 320 to rotate.

The shape of the cross-section of the magnetic core 410 is substantially triangular prism-shaped, and an edge of each magnetic core 410 faces an axis of the rotating shaft 210. For example, the edges of the magnetic core 410 are rounded, that is, the edges of the magnetic core 410 are relatively smooth and blunt rounded edges, so as to eliminate the sharp edges on the magnetic core 410, which not only facilitates the subsequent winding of the coil 420, but also facilitates the protection of the insulating material coated on the coil 420. In another example, the cross-sectional shape of the magnetic core 410 can also be a sector, a circle, a trapezoid, a sector ring, etc.

It should be understood that the structure of the magnetic core 410 is not limited to the above structure. In other embodiments, each magnetic core 410 includes a magnetic column and a head portion (i.e., a pole shoe) provided on the magnetic column. Two head portions are provided, both ends of the magnetic column are provided with the head portion. An extending direction of the magnetic column is consistent with the extending direction of the rotating shaft 210, and the coil 420 is wound on the magnetic column of each magnetic core 410. In the extending direction of the magnetic column, a size of the cross-section of the magnetic column remains constant. In general, a thickness of the magnetic column is uniform. At this time, the first rotor unit 310 and the second rotor unit 320 are provided adjacent to the two head portions respectively.

However, the columnar magnetic core 410 shown in FIG. 2, FIG. 4, FIG. 8, and FIG. 9 has no head portion (i.e. pole shoe) with large width. In this case, the entire magnetic core 410 can be magnetically coupled with the rotor 300. Compared with the magnetic core 410 having the head portion, on the one hand, the magnetic core 410 having only the columnar shape can reduce the magnetic loss, and increase the magnetic coupling density between the magnetic core 410 and the rotor 300 to increase the torque of the stator 400 to the rotor 300 under the same current. On the other hand, the magnetic core 410 without the head portion can also greatly reduce the power reduction problem of the driving device 20 due to the local magnetic short circuit caused by the contact between the adjacent magnetic cores 410.

Specifically, the magnetic core 410 is made of a magnetic material, such as silicon steel. Therefore, the magnetic core 410 is attractive to the first magnetic member 311 and the second magnetic member 321 at the same time, that is, an attractive force is formed between the stator 400 and the first rotor unit 310, and an attractive force is formed between the stator 400 and the second rotor unit 320.

Specifically, the stator 400 further includes a positioning member 430, the positioning member 430 is fixedly connected to the plurality of magnetic cores 410, and the positioning member 430 separates the adjacent magnetic cores 410 to prevent the adjacent magnetic cores 410 from coming into contact. The positioning member 430 is made of polyetheretherketone or ceramic. Both polyetheretherketone and ceramic are non-conductive and non-magnetic materials, so that the performance of the stator 400 is not affected, and the polyetheretherketone has the advantages of hydrolysis resistance, dimensional stability, electrical performance, insulating performance, etc. The ceramic has high biocompatibility, high mechanical strength, good wear resistance and corrosion resistance.

Specifically, the housing assembly 100 further includes a support member 130. The support member 130 is a shaft sleeve or a bearing. The support member 130 is mounted in the receiving cavity 101 and is fixedly connected to the housing 110. An end of the rotating shaft 210 away from the connecting section 211 is mounted on the support member 130. Specifically, both the rotor 300 and the stator 400 are located between the shaft sleeve 120 and the support member 130. In the illustrated embodiment, the support member 130 is a shaft sleeve.

In the illustrated embodiment, the housing assembly 100 further includes a fixing member 140 mounted in the receiving cavity 101. The fixing member 140 is provided with a mounting cavity 142 and a fluid channel in communication with the mounting cavity 142, the support member 130 is fixedly accommodated in the mounting cavity 142, and the fluid channel is configured to be in communication with the flushing line.

The aforementioned driving device 20 and the blood pump 1 have at least the following advantages.

According to the aforementioned driving device 20 and the blood pump 1, since the mounting hole 102 of the housing assembly 100 is provided with the concave spherical wall 103 and the rotating head 220 fixedly connected to the rotating shaft 210 is provided with the convex spherical surface 222 protruding along the axis of the rotating shaft 210 in a direction away from the connecting section 211 of the rotating shaft 210 configured to be connected to the impeller 40, the convex spherical surface 222 abuts against the concave spherical wall 103 through the magnetic force between the rotor 300 and the stator 400, which not only realizes axial limiting of the rotating shaft 210, but also realizes radial limiting of the rotating shaft 210, so that the structure of the driving device 20 is simpler and more compact, which greatly reduces assembly difficulty of the driving device 20.

Moreover, since the convex spherical surface 222 of the rotating head 220 protrudes along the axis of the rotating shaft 210 in a direction away from the connecting section 211 of the rotating shaft 210 configured to be connected to the impeller 40 and the concave spherical wall 103 of the mounting hole 102 is configured to abut against and fit with the convex spherical surface 222, the concave spherical wall 103 of the mounting hole 102 is recessed from the outside toward the inside of the receiving cavity 101 of the housing assembly 100. With such structural design, when assembling the driving device 20 the impeller 40 is fixedly connected to the rotating shaft 210 before the rotating shaft 210 is mounted to the housing assembly 100, so as to directly detect and adjust coaxiality of the impeller 40 and the rotating shaft 210, and then the rotating shaft 210 fixedly connected to the impeller 40 is assembled to the housing assembly 100 from the mounting hole 102. In this way, the rotating shaft 210 of the driving device 20 and the impeller 40 can have high coaxiality, which helps to reduce difficulty of assembly, improve production efficiency and yield of the driving device 20 and the blood pump 1, and improve reliability of the driving device 20 and the blood pump 1. However, due to the structural design of the driving device of the conventional blood pump, for example, the convex spherical surface 222 protrudes towards the connecting section 211 of the rotating shaft 210 and the concave spherical wall 103 is recessed towards the connecting section 211 of the rotating shaft 210, when assembling the driving device of the conventional blood pump, the rotating shaft 210 can only be mounted on the housing assembly 100 first, and then the impeller 40 is mounted on the rotating shaft 210. In this way, it is difficult to ensure the coaxiality of the rotating shaft 210 and the impeller 40, which increases the difficulty of assembly, resulting in low production efficiency and yield of the driving device and the blood pump, and affecting the reliability of the driving device and the blood pump.

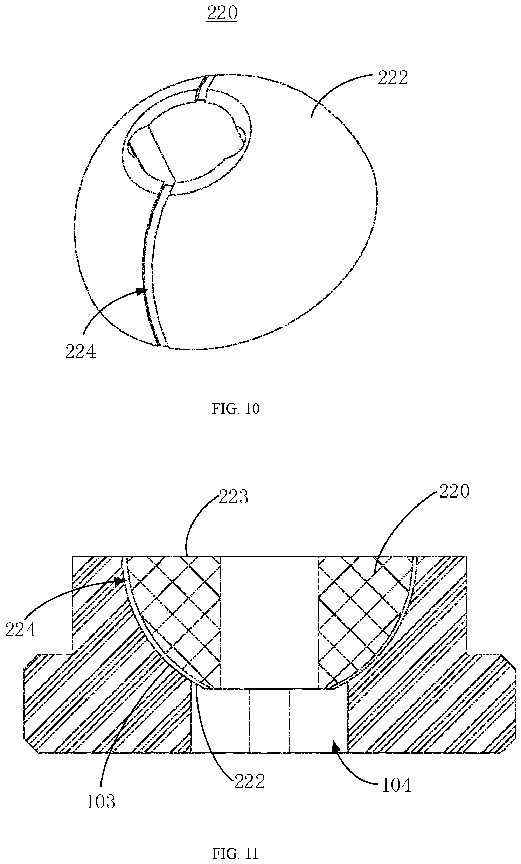

As shown in FIG. 10 and FIG. 11, a driving device of a blood pump according to a second embodiment is substantially the same as that of the driving device 20 according to the first embodiment, and a difference lies in that the structure of the rotating head 220 is different.

A portion of the convex spherical surface 222 of the rotating head 220 in this embodiment is recessed to form a flow guide groove 224. The flow guide groove 224 extends from the bottom surface 223 of the rotating head 220 along the convex spherical surface 222 in a direction away from the bottom surface 223, and the flow guide groove 224 is in communication with the receiving cavity of the housing assembly through the communication hole 104. Specifically, the flow guide groove 224 extends from the bottom surface 224 to the top surface 225 along the convex spherical surface 222.

The arrangement of the flow guide groove 224 helps to improve smoothness of the flow of the flushing fluid. At the same time, the arrangement of the flow guide groove 224 can also allow the flushing fluid to better enter between the concave spherical wall 103 of the mounting hole 102 and the convex spherical surface 224 of the rotating head 220 to serve as a lubrication, so as to reduce the wear between the concave spherical wall 103 of the mounting hole 102 and the convex spherical surface 224 of the rotating head 220.

Moreover, since the structure of the driving device of the second embodiment is substantially the same as that of the driving device 20 of the first embodiment, the driving device of the second embodiment also has a similar effect to that of the driving device 20 of the first embodiment, which is not described herein again.

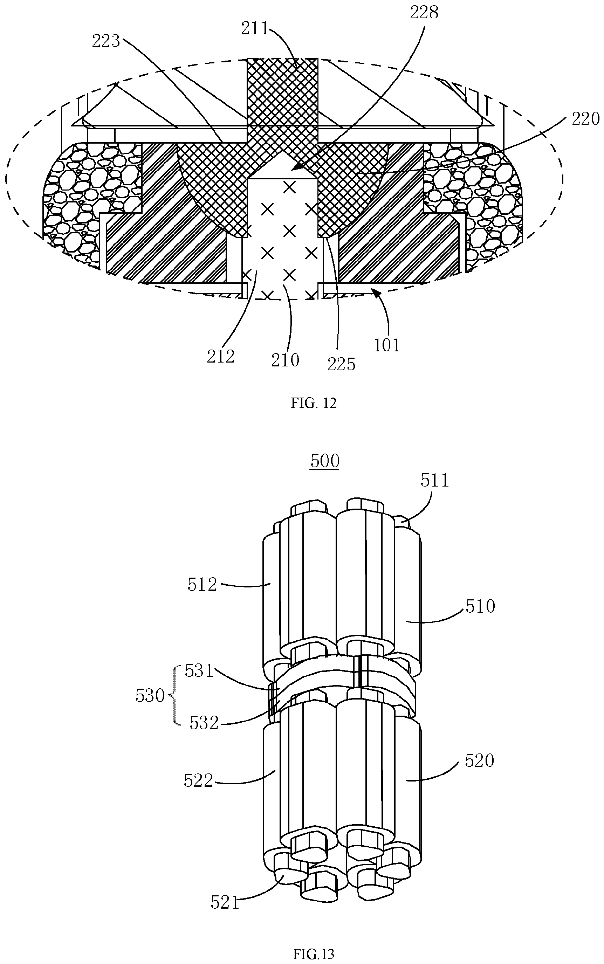

As shown in FIG. 12, a driving device of a blood pump according to a third embodiment is substantially the same as that of the driving device 20 according to the first embodiment, and a difference lies in that the structure of the rotating component 200 is different.

In this embodiment, an end of the connecting section 211 of the rotating shaft 210 is fixedly connected to the bottom surface 223 of the rotating head 210, and an axis of the connecting section 211 passes through a center of the bottom surface 223 of the rotating head 220 and is perpendicular to the bottom surface 223. The rotating shaft 210 further includes a built-in section 212 separated from the connecting section 211. The built-in section 212 and the connecting section 211 are arranged coaxially, and the built-in section 212 is fixedly connected to the rotating head 220.

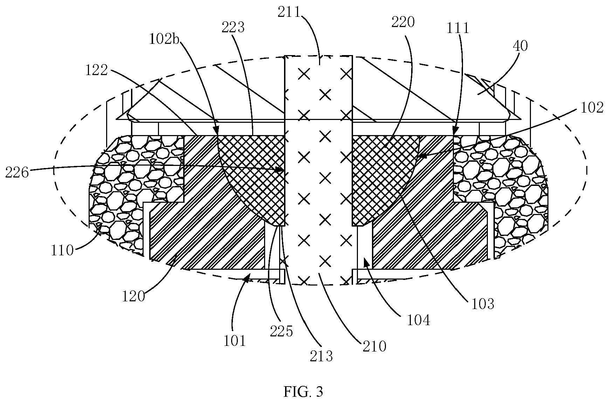

The rotor is fixedly connected to the built-in section 212 of the rotating shaft 210. In the illustrated embodiment, the rotating head 220 is provided with an assembly hole 228 extending from the top surface 225 of the rotating head 220 towards the bottom surface 223. A center axis of the assembly hole 228 passes through a center of the top surface 225 and the center of the bottom surface 223. That is, in this embodiment, the assembly hole 228 replaces the through hole 226 shown in FIG. 3. One end of the built-in section 212 is accommodated in the assembly hole 228, and the other end of the built-in section 212 extends into the receiving cavity 101, for example, it is mounted on the support member.

Moreover, since the structure of the driving device of the third embodiment is substantially the same as that of the driving device 20 of the first embodiment, the driving device of the third embodiment also has a similar effect to that of the driving device 20 of the first embodiment, which is not described herein again.

A structure of a driving device of the blood pump according to a fourth embodiment is substantially the same as that of the driving device 20 according to the first embodiment, and a difference lies in that the structure of the stator is different.

As shown in FIG. 13, in this embodiment, a stator 500 includes a first stator unit 510, a second stator unit 520, and a magnetic conduction member 530. The first stator unit 510, the magnetic conduction member 530, and the second stator unit 520 are arranged sequentially along the axis of the rotating shaft. The rotating shaft is rotatably provided through the first stator unit 510, the magnetic conduction member 530, and the second stator unit 520.

The first stator unit 510, the second stator unit 520, and the magnetic conduction member 530 are located between the first rotor unit 310 and the second rotor unit 320 shown in FIG. 4 and FIG. 8. The first stator unit 510 is provided adjacent to the first rotor unit 310, and the second stator unit 520 is provided adjacent to the second rotor unit 320. The first stator unit 510 can drive the first rotor unit 310 to rotate, and the second stator unit 520 can drive the second rotor unit 320 to rotate. The first attractive force is formed between the first stator unit 510 and the first rotor unit 310, and the second attractive force is formed between the second stator unit 520 and the second rotor unit 320.

Structures of the first stator unit 510 and the second stator unit 520 are substantially the same as the structure of the stator 400 shown in FIG. 4. The first stator unit 510 includes a first magnetic core 511 and a first coil 512, and the first coil 512 is wound on the first magnetic core 511. The second stator unit 520 includes a second magnetic core 521 and a second coil 522 wound on the second magnetic core 521. The first magnetic core 511 and the second magnetic core 521 may be provided with head portions, respectively, or may not be provided with head portions. A difference lies in that the first stator unit 510 and the second stator unit 520 are not provided with the positioning member 430 shown in FIG. 4.

The first magnetic core 511 of the first stator unit 510 and the second magnetic core 521 of the second stator unit 520 are both fixedly connected to the magnetic conduction member 530. In the case of the first magnetic core 511 is provided with the head portion, an end of the first magnetic core 511 away from the head portion is fixedly connected to the magnetic conduction member 530, and the first rotor unit 310 is provided adjacent to the head portion of the first magnetic core 511. In the case of the second magnetic core 521 is provided with the head portion, an end of the second magnetic core 521 away from the head portion is fixedly connected to the magnetic conduction member 530, and the second rotor unit 320 is provided adjacent to the head portion of the second magnetic core 521. In the case of the first magnetic core 511 is not provided with the head portion, one end of the first magnetic core 511 is fixedly connected to the magnetic conduction member 530, and the first rotor unit 310 is provided adjacent to the other end of the first magnetic core 511. In the case of the second magnetic core 521 is not provided with the head portion, one end of the second magnetic core 521 is fixedly connected to the magnetic conduction member 530, and the second rotor unit 320 is provided adjacent to the other end of the second magnetic core 521.

The magnetic conduction member 530 serves to close a magnetic circuit, so as to promote and increase generation of the magnetic flux and improve a coupling capability. Therefore, both the first magnetic core 511 of the first stator unit 510 and the second magnetic core 521 of the second stator unit 520 are fixedly connected to the magnetic conduction member 530, which can close a magnetic circuit between the first stator unit 510 and the first rotor unit 310, and close a magnetic circuit between the second stator unit 520 and the second rotor unit 320, so as to increase the magnetic flux. Therefore, the arrangement of the magnetic conduction member 530 helps reducing an overall diameter of the driving device 20. In addition, both the first magnetic core 511 of the first stator unit 510 and the second magnetic core 521 of the second stator unit 520 are fixedly connected to the magnetic conduction member 530, so that the first stator unit 510 and the second stator unit 520 can be positioned and mounted, and the assembly difficulty of the first stator unit 510 and the second stator unit 520 is reduced. In order to facilitate fixation of the stator 500 in the housing assembly, a slot cooperating with the magnetic conduction member 530 may be provided in the housing of the housing assembly, and the slot is engaged with the magnetic conduction member 530 to achieve overall fixation of the stator 500. Therefore, the magnetic conduction member 530 provided in the above manner can also reduce arrangement of a positioning structure in the housing assembly, thereby simplifying the structure of the housing assembly and simplifying the assembly process of the entire driving device.

Specifically, the magnetic conduction member 530 includes a first magnetic conduction plate portion 531 and a second magnetic conduction plate portion 532. The first magnetic conduction plate portion 531 is fixedly connected to the first magnetic core 511 of the first stator unit 510, and the second magnetic conduction plate portion 532 is fixedly connected to the second magnetic core 521 of the second stator unit 520. The first magnetic conduction plate portion 531 and the second magnetic conduction plate portion 532 are stacked. That is, sides of the first magnetic conduction plate portion 531 and the second magnetic conduction plate portion 532 adjacent to each other abut against each other. The rotating shaft is rotatably provided through the first magnetic conduction plate portion 531 and the second magnetic conduction plate portion 532.

Specifically, the first magnetic conduction plate portion 531 is fixedly connected to the second magnetic conduction plate portion 532, so that the first stator unit 510, the second stator unit 520, and the magnetic conduction member 530 formed as a whole and assembled into the housing, so that the assembly of the stator 500 is easier.

Specifically, the first magnetic conduction plate portion 531 and the second magnetic conduction plate portion 532 are fixed by welding or adhesion. In other words, the first magnetic conduction plate portion 531 and the second magnetic conduction plate portion 532 are two separated components prior to assembly. By arranging the magnetic conduction member 530 into the first magnetic conduction plate portion 531 and the second magnetic conduction plate portion 532 that are separated prior to assembly, when assembling the driving device, the first magnetic core 511 may be fixedly connected to the first magnetic conduction plate portion 531, the second magnetic core 521 may be fixedly connected the second magnetic conduction plate portion 532, and then the first magnetic conduction plate portion 531 and the second magnetic conduction plate portion 532 are stacked and fixed. In this way, the first magnetic core 511 and the second magnetic core 521 can be easily assembled to the first magnetic conduction plate portion 531 and the second magnetic conduction plate portion 532, respectively, and the first magnetic core 511 and the second magnetic core 521 can be assembled more easily.

It should be noted that the first magnetic conduction plate portion 531 and the second magnetic conduction plate portion 532 are made of silicon steel, and the first magnetic core 511 and the second magnetic core 521 are made of silicon steel. The magnetic conduction member 530 is not limited to the above manner in which it is formed by combining the first magnetic conduction plate portion 531 and the second magnetic conduction plate portion 532 that are separated prior to assembly. In some embodiments, the first magnetic conduction plate portion 531 and the second magnetic conduction plate portion 532 are not fixedly connected together, but stacked together. In some embodiments, the magnetic conduction member 530 may be an integrally formed plate-like structure. Both the first magnetic core 511 and the second magnetic core 521 are connected to the magnetic conduction member 530, that is, the first stator unit 510 and the second stator unit 520 share one magnetic conduction member 530.

Since the structure of the driving device of the fourth embodiment is substantially the same as that of the driving device 20 of the first embodiment, the driving device of the fourth embodiment also has a similar effect to that of the driving device 20 of the first embodiment, which is not described herein again.

A structure of a driving device of a blood pump according to a fifth embodiment is substantially the same as that of the driving device 20 according to the first embodiment, and a difference lies in that the structure of the rotor is different.

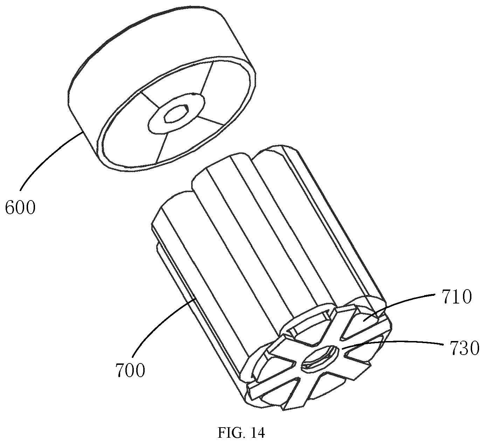

As shown in FIG. 14, in this embodiment, a rotor 600 is provided with a rotor unit, and a structure of the rotor 600 is the same as the structure and the arrangement manner of the first rotor unit 310 in FIG. 2, FIG. 4, FIG. 8, and FIG. 9 in the first embodiment. Along the axis of the rotating shaft, the rotor 600 is located between the rotating head and a stator 700, in other words, the rotor 600 is located between the shaft sleeve and the stator 700. An attractive force is formed between the rotor 600 and the stator 700, and the convex spherical surface of the rotating head abuts against the concave spherical wall of the mounting hole under an action of the attractive force.

In the illustrated embodiment, the structure of the stator 700 is the same as that of the stator 400 in FIG. 2, FIG. 4, FIG. 8, and FIG. 9. It should be understood that the structure of the stator 700 may alternatively be different from that of the stator 400 in FIG. 2, FIG. 4, FIG. 8, and FIG. 9. In some embodiments, the stator 700 does not have the positioning member 730, but has a magnetic conduction back plate (not shown), and the magnetic conduction back plate is fixedly connected to an end of a magnetic core 710 away from the rotor 600. The magnetic conduction back plate is configured to close a magnetic circuit, so as to promote and increase generation of magnetic flux, improve the coupling capability, and increase the magnetic flux, which helps to reduce the overall diameter of the driving device. The magnetic core 710 may have a head portion (a pole shoe) or does not have the head portion. When the magnetic core 710 has the head portion, the head portion is located at an end of the magnetic core 710 adjacent to the rotor 600.

Since the structure of the driving device of the fifth embodiment is substantially the same as that of the driving device 20 in the first embodiment, the driving device of the fifth embodiment also has a similar effect to that of the driving device 20 of the first embodiment, which is not described herein again.

A structure of a driving device of a blood pump according to a sixth embodiment is substantially the same as that of the driving device according to the fifth embodiment, and a difference lies in that the structure of the rotating shaft is different.

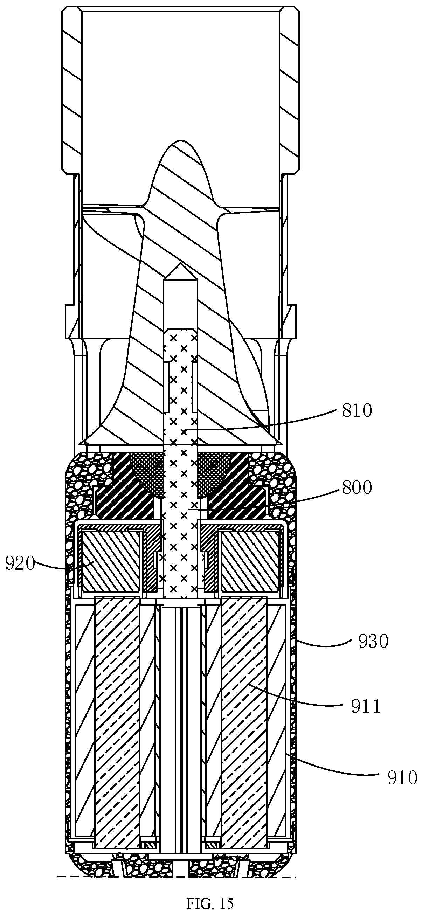

As shown in FIG. 15, in this embodiment, an end of a rotating shaft 800 away from a connecting section 810 is spaced apart from a stator 910. That is, the rotating shaft 800 is not provided in the stator 910, and the rotating shaft 800 is located outside the stator 910. A rotor 920 is fixedly connected to an end of the rotating shaft 800 away from the connecting section 810.

When a cross-sectional area of a magnetic core 911 of the stator 910 is larger, magnetic flux generated is larger, torque of the stator 910 to the rotor 920 is larger, and a required current is smaller, which helps to reduce power consumption and reduce heat generation. Since the rotating shaft 800 is not provided in the stator 910, occupation of a mounting space of the magnetic core 911 by the rotating shaft 800 can be prevented, which helps to increase a size of a cross-sectional of the magnetic core 911 to increase driving torque of the stator 910 to the rotor 920 while keeping an outer diameter of a housing assembly 930 (specifically, a housing) unchanged. When required torque is the same, in this manner, a current supply to the stator 910 can be reduced, thereby reducing power consumption, also reducing heat generation of the driving device, and the blood pump is prevented from causing discomfort or even harm to the human body due to excessive temperature caused by heat accumulation during operation.

In this embodiment, the support member 130 and the fixing member 140 similar to those in FIG. 2 and FIG. 4 may be omitted. Similarly, in this embodiment, the structure of the stator 910 may be the same as that of the stator 400 in FIG. 2, FIG. 4, FIG. 8, and FIG. 9 and may alternatively be different from that of the stator 400 in FIG. 2, FIG. 4, FIG. 8, and FIG. 9. For example, in some embodiments, the stator 910 does not have the positioning member 430 in FIG. 4 and FIG. 8, but has a magnetic conduction back plate (not shown), and the magnetic conduction back plate is fixedly connected to an end of the magnetic core 911 away from the rotor 920. The magnetic conduction back plate is configured to close a magnetic circuit, so as to promote and increase generation of magnetic flux, improve the coupling capability, and increase the magnetic flux, which helps to reduce the overall diameter of the driving device.

Since the structure of the driving device of the sixth embodiment is substantially the same as that of the driving device of the fifth embodiment, the driving device of the sixth embodiment also has a similar effect to that of the driving device of the fifth embodiment, which is not described herein again.

The foregoing embodiments are merely intended to describe the technical solutions of the present disclosure, but not to limit the present disclosure. Although the present disclosure is described in detail with reference to the foregoing embodiments, those of ordinary skill in the art should understand that they may still make modifications to the technical solutions described in the foregoing embodiments or make equivalent replacements to some technical features thereof, without departing from the spirit and scope of the technical solutions of the embodiments of the present disclosure, all of which fall within the protection scope of the present disclosure.

## Claims

1. A driving device configured to driving an impeller to rotate, the driving device comprising:
a housing assembly provided with a mounting hole, the mounting hole being provided with a concave spherical wall;
a rotating component comprising a rotating shaft and a rotating head fixedly connected to the rotating shaft, the rotating shaft being rotatably mounted on the housing assembly, and the rotating shaft being provided with a connecting section located outside the housing assembly and configured to be connected to the impeller, the rotating head being rotatably provided in the mounting hole, the rotating head being provided with a convex spherical surface, the convex spherical surface protruding in a direction away from the connecting section along an axis of the rotating shaft, and the convex spherical surface being capable of abutting against the concave spherical wall;
a rotor fixedly connected to the rotating shaft; and
a stator configured to drive the rotor to rotate, a magnetic force forming between the rotor and the stator, and the magnetic force configured to enable the convex spherical surface to abut against the concave spherical wall.

2. The driving device according to claim 1, wherein the rotor is located between the rotating head and the stator along the axis of the rotating shaft, and an attractive force is formed between the rotor and the stator, and the convex spherical surface abuts against the concave spherical wall under an action of the attractive force.

3. The driving device according to claim 1, wherein the rotor comprises a first rotor unit and a second rotor unit, the rotating head, the first rotor unit, the stator, and the second rotor unit are arranged sequentially along the axis of the rotating shaft, the stator is capable of driving the first rotor unit and the second rotor unit to rotate, a first attractive force is formed between the stator and the first rotor unit, a second attractive force is formed between the stator and the second rotor unit, the first attractive force is greater than the second attractive force, and the convex spherical surface abuts against the concave spherical wall under a combined action of the first attractive force and the second attractive force.

4. The driving device according to claim 3, wherein the stator comprises a plurality of magnetic cores, a plurality of coils, and a positioning member, the plurality of magnetic cores are spaced apart in a circle, the plurality of coils are respectively wound around the plurality of the magnetic cores, the positioning member is fixedly connected to the plurality of magnetic cores, the positioning member separates the adjacent magnetic cores, and the positioning member is made of polyetheretherketone or ceramic.

5. The driving device according to claim 1, wherein the stator comprises a plurality of magnetic cores, a plurality of coils, and a magnetic conduction member, the plurality of magnetic cores are spaced apart in a circle, the plurality of coils are respectively wound around the plurality of the magnetic cores, the magnetic cores are fixedly connected to the magnetic conduction member, and an inner wall of the housing assembly is provided with a slot engaging with the magnetic conduction member.

6. The driving device according to claim 5, wherein the stator comprises a first stator unit and a second stator unit, the rotor comprises a first rotor unit and a second rotor unit, the rotating head, the first rotor unit, the first stator unit, the magnetic conduction member, the second stator unit, and the second rotor unit are arranged sequentially along the axis of the rotating shaft, a first attractive force is formed between the first stator unit and the first rotor unit, a second attractive force is formed between the second stator unit and the second rotor unit, the first attractive force is greater than the second attractive force, and the convex spherical surface abuts against the concave spherical wall under a combined action of the first attractive force and the second attractive force; and
the magnetic conduction member comprises a first magnetic conduction plate portion and a second magnetic conduction plate portion that are separately arranged and stacked together, the first magnetic conduction plate portion is fixedly connected to the magnetic core of the first stator unit, the second magnetic conduction plate portion is fixedly connected to the magnetic core of the second stator unit, and the rotating shaft rotatably extends through the first magnetic conduction plate portion and the second magnetic conduction plate portion.

7. The driving device according to claim 1, wherein the housing assembly is further provided with a receiving cavity in communication with the mounting hole, the rotor and the stator are mounted in the receiving cavity, the mounting hole is provided with a first opening and a second opening opposite to the first opening, the first opening is in communication with the receiving cavity, a diameter of the mounting hole gradually increases from the first opening to the second opening, the concave spherical wall extends from the first opening to the second opening, the rotating head is further provided with a bottom surface connected to the convex spherical surface, and when the convex spherical surface abuts against the concave spherical wall, the bottom surface is coplanar with a plane where the second opening is located.

8. The driving device according to claim 7, wherein a ratio of a diameter of the first opening to a diameter of the bottom surface of the rotating head is 0.3 to 0.6;
and/or a portion of the convex spherical surface is recessed to form a flow guide groove, the flow guide groove extends from the bottom surface of the rotating head along the convex spherical surface in a direction away from the bottom surface, and the flow guide groove is in communication with the receiving cavity.

9. The driving device according to claim 1, wherein the housing assembly is further provided with a receiving cavity in communication with the mounting hole, and the rotor and the stator are mounted in the receiving cavity, the mounting hole is provided with a first opening and a second opening opposite to the first opening, the first opening is in communication with the receiving cavity, a diameter of the mounting hole gradually increases from the first opening to the second opening, and the concave spherical wall extends from the first opening to the second opening; and
a width of the rotating head in a direction perpendicular to an axial direction of the rotating shaft is less than a diameter of the second opening.

10. The driving device according to claim 9, wherein a gap between a hole wall of the mounting hole at the second opening and the rotating head is less than or equal to 2 microns.

11. The driving device according to claim 1, wherein the housing assembly is further provided with a receiving cavity and a communication hole in communication with the mounting hole and the receiving cavity, a diameter of the mounting hole gradually increases in a direction away from the communication hole, the rotating shaft rotatably extends through the communication hole, and a hole wall of the communication hole is partially recessed to form a drainage groove extending along an axis of the communication hole, the drainage groove is in communication with both the receiving cavity and the mounting hole, and the rotor and the stator are mounted in the receiving cavity.

12. The driving device according to claim 1, wherein the housing assembly comprises a housing and a shaft sleeve, the housing is provided with a receiving cavity and a communication port in communication with the receiving cavity, the shaft sleeve is mounted in the receiving cavity, the shaft sleeve is provided with an outer surface coplanar with a plane where the communication port is located, the mounting hole is provided on the shaft sleeve, the mounting hole is provided with a first opening and a second opening opposite to the first opening, the first opening is in communication with the receiving cavity, and a plane where the second opening is located is coplanar with the outer surface of the shaft sleeve.

13. The driving device according to claim 1, wherein the rotating head is further provided with a bottom surface connected to the convex spherical surface, the rotating shaft fixedly extends through the rotating head, and the axis of the rotating shaft passes through a center of the bottom surface and is perpendicular to the bottom surface;
or the rotating head is further provided with a bottom surface connected to the convex spherical surface, an end of the connecting section is fixedly connected to the bottom surface, an axis of the connecting section passes through the center of the bottom surface and is perpendicular to the bottom surface, the rotating shaft further comprises a built-in section separated from the connecting section, the built-in section and the connecting section are arranged coaxially, the built-in section is fixedly connected to the rotating head, and the rotor is fixedly connected to the built-in section.

14. The driving device according to claim 13, wherein the rotating head is further provided with a top surface, the top surface and the bottom surface are spaced apart along the axis of the rotating shaft and provided in parallel, the rotating head is provided with an assembly hole extending from the top surface towards the bottom surface, a center axis of the assembly hole passes through a center of the top surface and the center of the bottom surface, an end of the built-in section is accommodated in the assembly hole, and another end of the built-in section protrudes outside the top surface.

15. The driving device according to claim 1, wherein the rotating head is further provided with a top surface, the top surface and the bottom surface are spaced apart along the axis of the rotating shaft, a diameter of the top surface is less than a diameter of the bottom surface, and the convex spherical surface connects the top surface and the bottom surface.

16. The driving device according to claim 15, wherein the housing assembly is further provided with a receiving cavity in communication with the mounting hole, and the rotor and the stator are mounted in the receiving cavity, the mounting hole is provided with a first opening and a second opening opposite to the first opening, the first opening is in communication with the receiving cavity, a diameter of the mounting hole gradually increases from the first opening to the second opening, the concave spherical wall extends from the first opening to the second opening, and the diameter of the top surface is less than a diameter of the first opening, so that the rotating head is partially located outside the mounting hole.

17. The driving device according to claim 15, wherein the rotating shaft is further provided with a limiting surface, and the top surface abuts against the limiting surface.

18. A blood pump comprising an impeller and a driving device, the driving device comprising:
a housing assembly provided with a mounting hole, the mounting hole being provided with a concave spherical wall;
a rotating component comprising a rotating shaft and a rotating head fixedly connected to the rotating shaft, the rotating shaft being rotatably mounted on the housing assembly, and the rotating shaft being provided with a connecting section located outside the housing assembly and fixedly connected to the impeller, the rotating head being rotatably provided in the mounting hole, the rotating head being provided with a convex spherical surface, the convex spherical surface protruding in a direction away from the connecting section along an axis of the rotating shaft, and the convex spherical surface being capable of abutting against the concave spherical wall;
a rotor fixedly connected to the rotating shaft; and
a stator configured to drive the rotor to rotate, a magnetic force forming between the rotor and the stator, and the magnetic force configured to enable the convex spherical surface to abut against the concave spherical wall.

19. The blood pump according to claim 18, further comprising a cannula assembly, a proximal end of the cannula assembly is provided with a liquid outlet, a distal end of the cannula assembly is provided with a liquid inlet, the impeller is rotatably provided in the cannula assembly, the proximal end of the cannula assembly is fixedly connected to a distal end of the driving device, and the cannula assembly comprises a tube body and a plurality of spaced inserting sheets extending from an end of the tube body along an axis of the tube body, and the liquid outlet is formed between two adjacent inserting sheets.

20. The blood pump according to claim 19, wherein the driving device is provided with a plurality of sinking grooves, ends of the inserting sheets away from the tube body are accommodated in the sinking grooves, and the plurality of the sinking grooves are in one-to-one correspondence with the plurality of inserting sheets.
